# EUROPEAN PATENT APPLICATION

(11) **EP 4 796 052 A1**
(43) Date of publication of application: **26.08.2026**
(21) Application number: 26160157.9
(22) Date of filing: 23.02.2026
(51) Int. Cl.: A61B 17/072

(54) **END EFFECTOR JAW CLOSURE FEATURES FOR SURGICAL STAPLER**

(30) Priority: 21.02.2025 US 202519060037
(71) Applicant: Cilag GmbH International, 6300 Zug (CH)
(72) Inventor: MAY, John P., Cincinnati, 45242 (US)
(74) Representative: Carpmaels & Ransford LLP

(57) **Abstract**

A first jaw (318) for use with an end effector (313) of a surgical instrument. A proximal end of the first jaw (318) including a ramp surface (352). The ramp surface including: a proximal engagement portion (A); an intermediate engagement portion (B), and a distal engagement portion (C). The intermediate engagement portion being distal to the proximal engagement portion, at least a portion of the proximal engagement portion being concavely curved. The distal engagement portion (C) being distal to the intermediate engagement portion. The ramp surface (352) being configured to be engaged by a translatable cam (333) of the surgical instrument to thereby pivot the first jaw (318) relative to a second jaw (316) of the end effector (313). The first (318) and second jaws (316) configured to cooperate to clamp and staple tissue (T) positioned therebetween.

## Description

### BACKGROUND

In some settings, endoscopic surgical instruments may be preferred over traditional open surgical devices to minimize the size of the surgical incision as well as post-operative recovery time and complications. Consequently, some endoscopic surgical instruments may be suitable for placement of a distal end effector at a desired surgical site through the cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft that extends proximally from the end effector to a handle portion, which is manipulated by the clinician, or alternatively to a robot. Such a shaft may enable insertion to a desired depth and rotation about the longitudinal axis of the shaft, thereby facilitating positioning of the end effector within the patient. Positioning of an end effector may be further facilitated through inclusion of one or more articulation joints or features, enabling the end effector to be selectively articulated or otherwise deflected relative to the longitudinal axis of the shaft.

Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Such endoscopic surgical staplers may also be used in open procedures and/or other non-endoscopic procedures. By way of example only, a surgical stapler may be inserted through a thoracotomy and thereby between a patient's ribs to reach one or more organs in a thoracic surgical procedure that does not use a trocar as a conduit for the stapler. Such procedures may include the use of the stapler to sever and close a vessel leading to an organ, such as a lung. For instance, the vessels leading to an organ may be severed and closed by a stapler before removal of the organ from the thoracic cavity. Of course, surgical staplers may be used in various other settings and procedures.

Such surgical staplers may include an end effector having a stationary first jaw and a pivotable second jaw that cooperate to close onto and thereby clamp tissue, and then staple the clamped tissue. However, for many such surgical staplers, the end effector is configured such that the pivotable second jaw advances through an initial portion of its closure stroke, where the end effector closure components are less mechanically advantaged, more quickly than through a final portion of the closure stroke, where the end effector closure components are more mechanically advantaged. Consequently, a relatively greater user input force is required to advance the end effector through the initial portion of the closure stroke, which results in undue stress on the end effector closure components. Additionally, these greater forces may encourage misalignment of the end effector closure components.

The features of the present disclosure seek to address the above deficiencies. In that regard, while various kinds of surgical instruments and associated components have been made and used, it is believed that no one prior to the inventor(s) has made or used the invention described in the appended claims.

### BRIEF DESCRIPTION OF THE DRAWINGS

The accompanying drawings, which are incorporated in and constitute a part of this specification, illustrate examples of the invention, and, together with the general description of the invention given above, and the detailed description of the examples given below, serve to explain the principles of the present invention.
FIG. 1 depicts a perspective view of an example of a surgical stapler;
FIG. 2 depicts a perspective view of an end effector of the surgical stapler of FIG. 1, shown in an open state;
FIG. 3 depicts an exploded perspective view of the end effector of FIG. 2;
FIG. 4A depicts a side cross-sectional view of the end effector of FIG. 2, taken along line 4-4 of FIG. 2, showing a firing beam and sled in a proximal undisplaced position;
FIG. 4B depicts a side cross-sectional view of the end effector of FIG. 2, taken along line 4-4 of FIG. 2, showing the firing beam and sled in a distal fired position;
FIG. 5 depicts an end cross-sectional view of the end effector of FIG. 2, taken along line 5-5 of FIG. 2 and omitting an upper anvil jaw, showing further details of a distal knife portion of the firing beam and the sled;
FIG. 6 depicts a perspective view of the end effector of FIG. 3, shown after having been fired once on a first section of tissue and being positioned to clamp and fire on a second section of tissue;
FIG. 7 depicts a perspective view of another illustrative end effector configured for use with the surgical stapler of FIG. 1;
FIG. 8 depicts a side elevational view of a pivoting anvil jaw of the end effector of FIG. 7;
FIG. 9 depicts a top plan view of the pivoting anvil jaw of FIG. 8;
FIG. 10 depicts a side elevational view of a closure ring of the end effector of FIG. 7;
FIG. 11 depicts a top plan view of the closure ring of FIG. 10;
FIG. 12A depicts a perspective view of the end effector of FIG. 7 during a first instant of time during closure;
FIG. 12B depicts a perspective view of the end effector of FIG. 7 during a second instant of time during closure;
FIG. 12C depicts a perspective view of the end effector of FIG. 7 fully closed;
FIG. 13 depicts a side elevational view of another illustrative end effector configured for use with the surgical stapler of FIG. 1, showing the end effector during a transition from a closed configuration to an open configuration;
FIG. 14 depicts a distally facing perspective side view of a closure ring positioned along a cartridge jaw of the end effector of FIG. 13;
FIG. 15 depicts a distally facing end view of the closure ring and the cartridge jaw of the end effector of FIG. 13;
FIG. 16 depicts a partial side cross-sectional view of the end effector of FIG. 13, showing the upper jaw and the cartridge jaw in the closed configuration, the upper jaw including a cam ramp surface having a proximal engagement portion, an intermediate engagement portion, and a distal engagement portion;
FIG. 17A depicts a partial side cross-sectional view of the end effector of FIG. 13 during distal translation of the closure ring along the proximal engagement portion of the cam ramp surface of FIG. 16;
FIG. 17B depicts a partial side cross-sectional view of the end effector of FIG. 13 during distal translation of the closure ring along the intermediate engagement portion of the cam ramp surface of FIG. 16; and
FIG. 17C depicts a partial side cross-sectional view of the end effector of FIG. 13 during distal translation of the closure ring along the distal engagement portion of the cam ramp surface of FIG. 16.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. In addition, the terms "upper," "lower," "lateral," "transverse," "bottom," "top," are relative terms to provide additional clarity to the figure descriptions provided below. The terms "upper," "lower," "lateral," "transverse," "bottom," "top," are thus not intended to unnecessarily limit the invention described herein.

Furthermore, the terms "about," "approximately," "substantially," and the like as used herein in connection with any numerical values, ranges of values, and/or geometric/positional quantifications are intended to encompass the exact value(s) or quantification(s) referenced as well as a suitable tolerance that enables the referenced feature or combination of features to function for the intended purpose described herein. For example, "substantially parallel" encompasses nominally parallel structures, and "substantially equal" values encompass nominally equal values.

### I. Illustrative Surgical Stapler

### A. Overview of Surgical Stapler Features

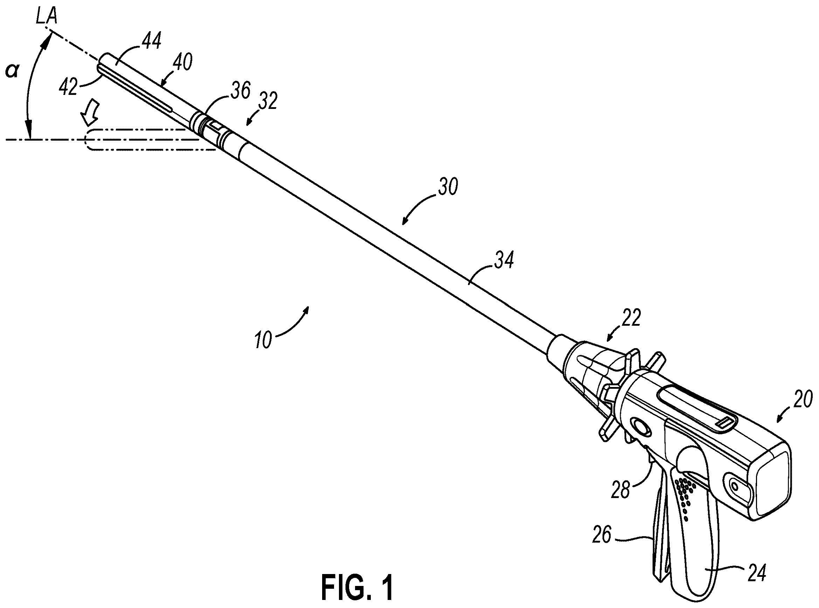

FIGS. 1-6 depict an illustrative surgical stapler (10) that is sized for insertion through a trocar cannula or a surgical incision (e.g., thoracotomy, etc.) to a surgical site in a patient for performing a surgical procedure. Surgical stapler (10) may also be referred to as a surgical instrument or simply as an instrument. Surgical stapler (10) includes a body exemplified as a handle assembly (20), a shaft (30) that extends distally from handle assembly (20) along a longitudinal axis (LA) and distally terminates at an articulation joint (32), and an end effector (40) operatively coupled with shaft (30) via articulation joint (32).

Once end effector (40) and articulation joint (32) are inserted distally through the cannula passageway of a trocar, articulation joint (32) may be remotely articulated, as depicted in phantom in FIG. 1, by an articulation control exemplified as a rotatable knob (22) of handle assembly (20), such that end effector (40) may be deflected from the longitudinal axis (LA) at a desired angle (α). Articulation joint (32) and related features for manipulating articulation joint (32) may be further configured in accordance with the teachings of U.S. Pat. No. 9,186,142, entitled "Surgical Instrument End Effector Articulation Drive with Pinion and Opposing Racks," issued on November 17, 2015, the disclosure of which is incorporated by reference herein in its entirety.

End effector (40) includes a cartridge jaw (42) configured to removably receive a staple cartridge (70) (also referred to as a "reload"), and an upper jaw exemplified as an anvil jaw (44) (also referred to as an "anvil") that pivots relative to cartridge jaw (42) to clamp tissue therebetween. In other versions, end effector (40) may be alternatively configured such that cartridge jaw (42) pivots relative to anvil jaw (44). Unless otherwise described, the term "pivot" (and variations thereof) as used herein in connection with the relative motion between jaws (42, 44) encompasses but is not necessarily limited to pivotal movement about a fixed axis. For instance, in some versions, anvil jaw (44) may pivot about an axis that is defined by a pin (or similar feature) that slidably translates along an elongate slot or channel as anvil jaw (44) moves toward cartridge jaw (42). Such translation may occur before, during, or after the pivotal motion. It should therefore be understood that such combinations of pivotal and translational movement are encompassed by the term "pivot" and variations thereof as used herein with reference to the relative motion between jaws (42, 44).

As shown in FIG. 1, handle assembly (20) includes a pistol grip (24) and a closure trigger (26). Closure trigger (26) is pivotable toward pistol grip (24) to cause clamping, or closing, of anvil jaw (44) toward cartridge jaw (42) of end effector (40). Such closing of anvil jaw (44) is provided through a closure tube (34) and a closure ring (36) of shaft (30), which both longitudinally translate relative to handle assembly (20) in response to pivoting of closure trigger (26) relative to pistol grip (24). Closure tube (34) extends along the length of shaft (30); and closure ring (36) is positioned distal to articulation joint (32). Articulation joint (32) is operable to transmit longitudinal movement from closure tube (34) to closure ring (36) to actuate anvil jaw (44) relative to cartridge jaw (42).

Handle assembly (20) also includes a firing trigger (28). An elongate actuator (not shown) extends longitudinally through shaft (30) and transmits a longitudinal firing motion from handle assembly (20) to a firing member (also referred to as a firing driver) exemplified as a firing beam (46) in response to actuation of firing trigger (28). As a result, firing beam (46) translates distally through a firing stroke to cause stapling and severing of tissue clamped by end effector (40), as will be described in greater detail below. Though not shown, handle assembly (20) may further include a motor operable to actuate such firing assembly components of surgical stapler (10) in response to actuation of firing trigger (28) by a user, for example as disclosed in U.S. Pat. No. 8,453,914, entitled "Motor-Driven Surgical Cutting Instrument with Electric Actuator Directional Control Assembly," issued June 4, 2013, the disclosure of which is incorporated by reference herein in its entirety.

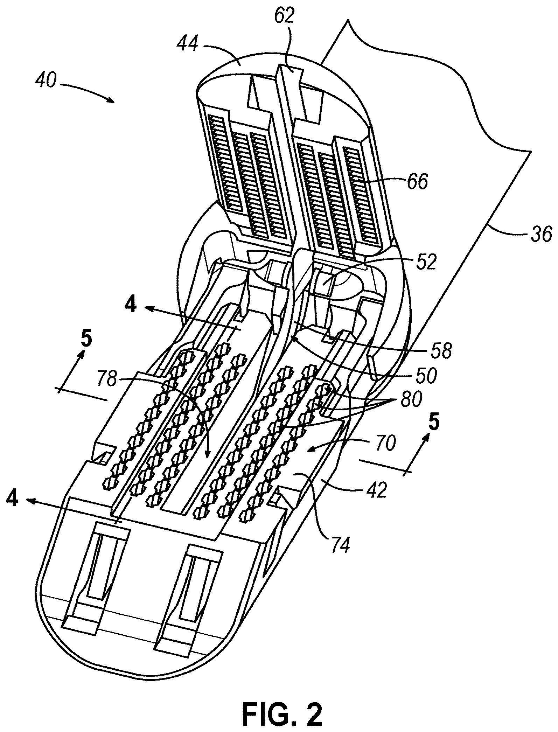

As shown in FIGS. 2-5, firing beam (46) includes a proximal beam portion (48) and a distal knife portion (50), where distal knife portion (50) may be integrally formed with a distal end of proximal beam portion (48), or separately formed and thereafter securely affixed to the distal end of proximal beam portion (48). Distal knife portion (50) includes a transversely oriented upper protrusion exemplified as an upper pin (52), a transversely oriented lower protrusion exemplified as a cap (54), a transversely oriented middle protrusion exemplified as a middle pin (56), and a distally presented cutting edge (58). Upper pin (52) is slidable within a longitudinal anvil jaw slot (62) of anvil jaw (44) and cap (54) is slidable along a lower surface of cartridge jaw (42) defined by a longitudinal cartridge jaw slot (64). Middle pin (56) is slidable along a top surface of cartridge jaw (42) and cooperates with cap (54) to stabilize and guide distal knife portion (50) along a longitudinal firing stroke. Firing beam (46) may be further configured and operable in accordance with the teachings of U.S. Pat. No. 9,717,497, entitled "Lockout Feature for Movable Cutting Member of Surgical Instrument," issued August 1, 2017, the disclosure of which is incorporated by reference herein in its entirety.

FIG. 2 shows anvil jaw (44) pivoted to an open state with firing beam (46) proximally positioned, which permits an unspent (i.e., unfired) staple cartridge (70) to be removably seated within a channel of cartridge jaw (42). As best seen in FIGS. 2-3, staple cartridge (70) includes a cartridge body (72) that presents an upper deck (74) defining a first stapling surface, and a lower pan (76) (also referred to as a "tray") coupled to an underside of cartridge body (72). A vertical knife slot (78) extends longitudinally through cartridge body (72) and is configured to slidably receive distal knife portion (50) of firing beam (46). In the present version, three rows of cartridge pockets (80) (also referred to as "staple openings," "staple apertures," or "staple cavities") are formed through upper deck (74) along each lateral side of knife slot (78).

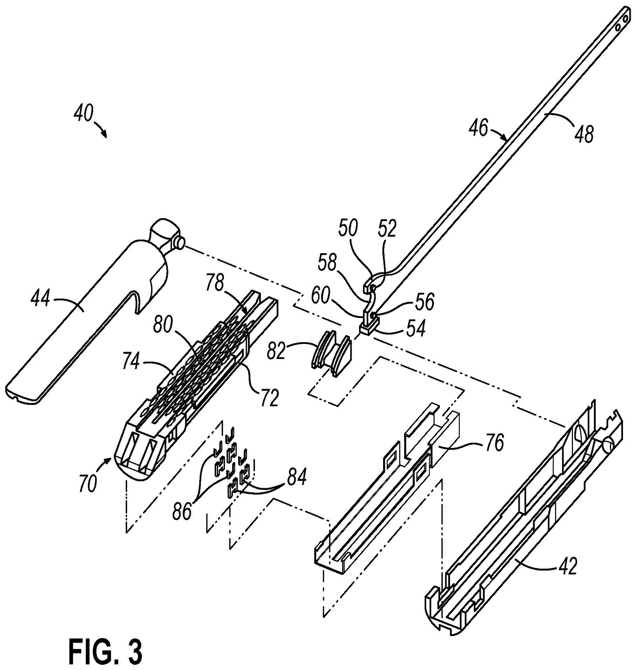

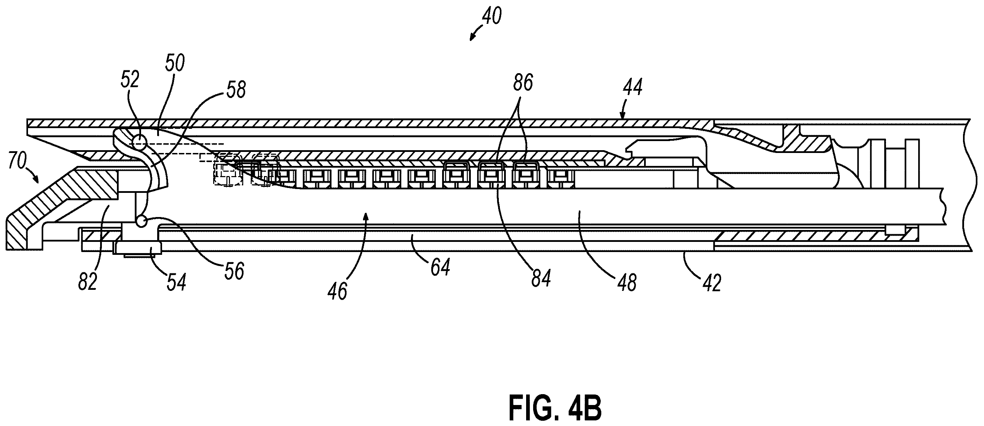

As shown in FIGS. 3-5, staple cartridge (70) further includes a sled (82) (also referred to as a "wedge sled") and a plurality of staple drivers (84) that are movably captured between cartridge body (72) and pan (76). Each staple driver (43) is aligned with and movable vertically within a respective cartridge pocket. Staples (86) are positioned within respective cartridge pockets (80) above respective staple drivers (84). During a firing stroke, sled (82) is actuated longitudinally within staple cartridge (70) by distal knife portion (50) from a proximal position shown in FIG. 4A to a distal position shown in FIG. 4B. Angled cam surfaces of sled (82) cam staple drivers (84) vertically upwardly within cartridge pockets (80) to drive staples (86) upwardly above deck (74), thereby ejecting staples (86) from cartridge pockets (80) and toward anvil jaw (44).

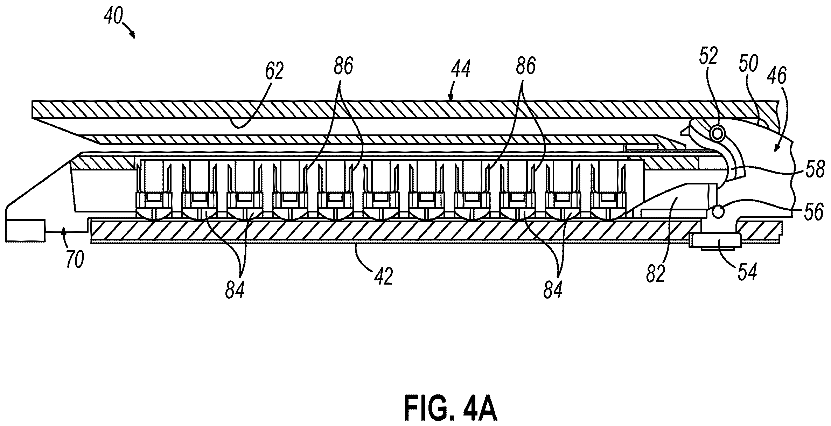

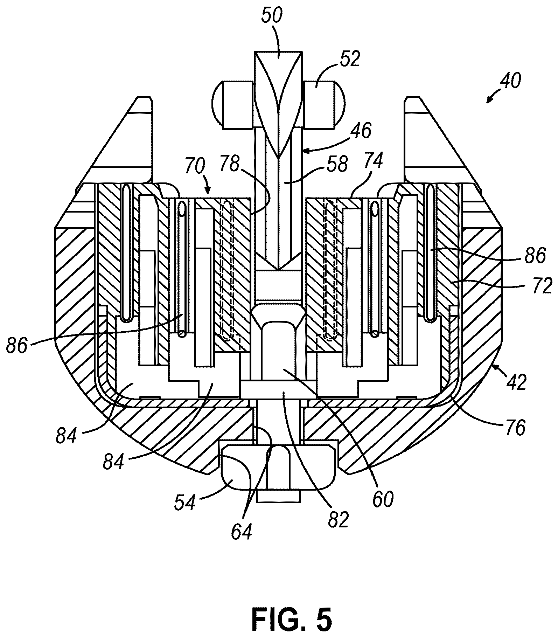

More specifically, with end effector (40) closed as shown in FIGS. 4A-4B, firing beam (46) is actuated distally into engagement with anvil jaw (44) by directing upper pin (52) into longitudinal anvil slot (62). A distal end projection (60) (see FIG. 5) of distal knife portion (50) of firing beam (46) engages a proximal end of sled (82) and drives sled (82) distally as distal knife portion (50) is advanced distally through staple cartridge (70) in response to actuation of firing trigger (28). During such firing, distal knife portion (50) advances distally along knife slot (78) of staple cartridge (70) so that cutting edge (58) severs tissue clamped between staple cartridge (70) and anvil jaw (44).

As shown in FIGS. 4A-4B, middle pin (56) and distal end projection (60) together actuate staple cartridge (70) by entering into knife slot (78), driving sled (82) into camming contact with staple drivers (84) to thereby actuate staple drivers (84) upwardly, which in turn drives staples (86) outwardly through cartridge pockets (80), through clamped tissue, and into forming contact with staple forming pockets (66) (see FIG. 2) on a second stapling surface defined by anvil jaw (44). Such stapling of tissue prompted by the camming interaction between sled (82) and staple drivers (84) is performed concurrently with the severing of tissue performed by cutting edge (58). However, it will be appreciated that for each longitudinal section of tissue clamped by end effector (40), staples (86) may be ejected into the tissue slightly before cutting edge (58) severs the tissue to ensure that the tissue is stapled and thus sealed before being severed. FIG. 4B depicts firing beam (46) fully distally translated at the end of a firing stroke after the tissue clamped by end effector (40) has been stapled and severed.

Staple cartridge (70) and anvil jaw (44) may be further configured and operable in accordance with the teachings of U.S. Pat. No. 9,808,A248, entitled "Installation Features for Surgical Instrument End Effector Cartridge," issued November 7, 2017; U.S. Pat. No. 9,839,421, entitled "Jaw Closure Feature for End Effector of Surgical Instrument," issued December 12, 2017; U.S. Pat. No. 10,092,292, entitled "Staple Forming Features for Surgical Stapling Instrument," issued October 9, 2018; and/or U.S. Pat. No. 10,130,359, entitled "Method for Forming a Staple," issued November 20, 2018, the disclosure of each of which is incorporated by reference herein in its entirety.

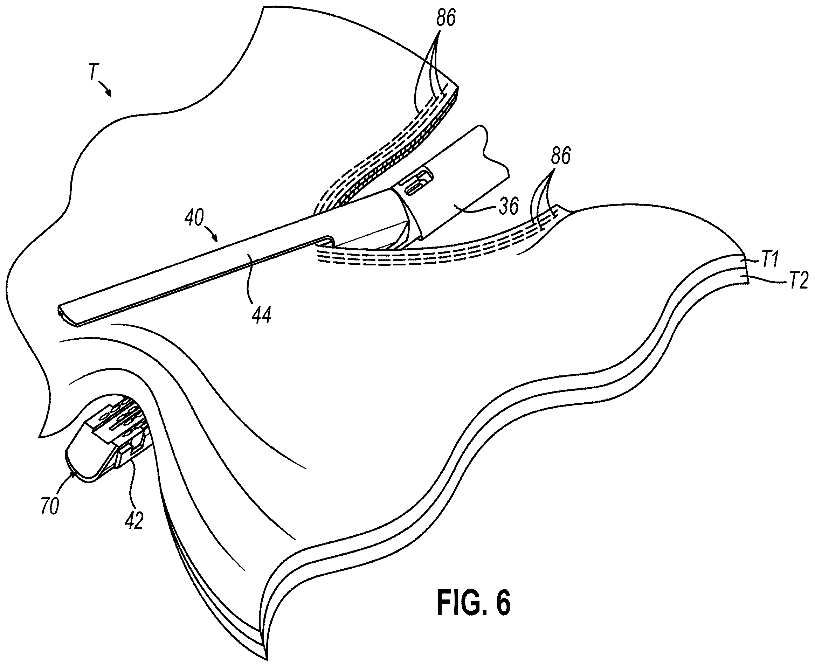

FIG. 6 shows end effector (40) having been actuated through a single firing stroke on tissue (T) having first and second layers (T1, T2). Cutting edge (58) (see FIGS. 2-5) has cut through tissue (T) while staple drivers (84) have driven three alternating rows of staples (86) through tissue (T) on each side of the cut line produced by cutting edge (58). After the first firing stroke is complete, end effector (40) is withdrawn from the patient, spent staple cartridge (70) is replaced with a new unspent staple cartridge (70), and end effector (40) is then again inserted into the patient to reach the stapling site for further cutting and stapling. This process may be repeated until the desired quantity and pattern of firing strokes across the tissue (T) has been completed.

Surgical stapler (10) described above as well as any of the illustrative surgical stapling features described below may be further configured in accordance with, or otherwise combined with, any of the teachings of U.S. Pat. App. No. 18/588,684, entitled "Method of Surgical Stapling," filed February 27, 2024, the disclosure of which is incorporated by reference herein in its entirety.

### B. Second Illustrative End Effector

As described above, closure tube (34) actuates closure ring (36) relative to cartridge jaw (42) and anvil jaw (44). Accordingly, geometries of each of closure ring (36), cartridge jaw (42), and anvil jaw (44) may affect the end effector closure rate and mechanical advantage when grasping tissue (T).

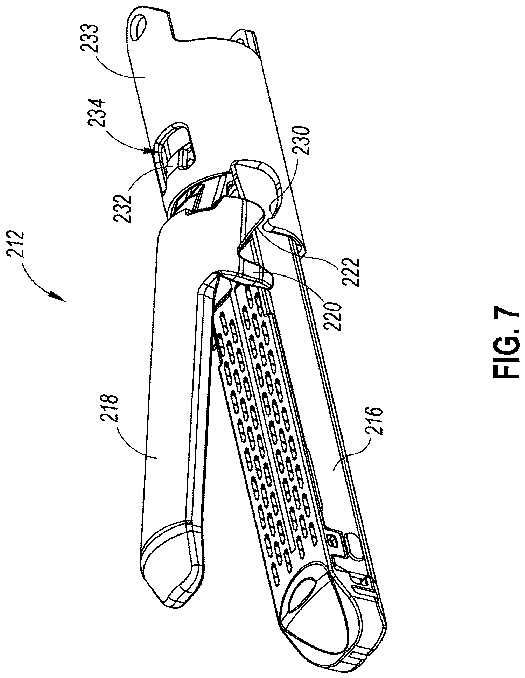

FIG. 7 shows an illustrative end effector (212) that may be readily incorporated into instrument (10). End effector (212) may be substantially similar in form and function to end effector (40). End effector (212) comprises a cartridge jaw (216) (also referred to as a cartridge jaw), a pivotable anvil (218) (also referred to as an anvil jaw), and a translatable cam in the form of a closure ring (233). Cartridge jaw (216) is similar to cartridge jaw (42) of end effector (40). Pivotable anvil (218) is similar to anvil jaw (44). Closure ring (233) of end effector (212) is similar to closure ring (36), except that closure ring (233) comprises extensions (230).

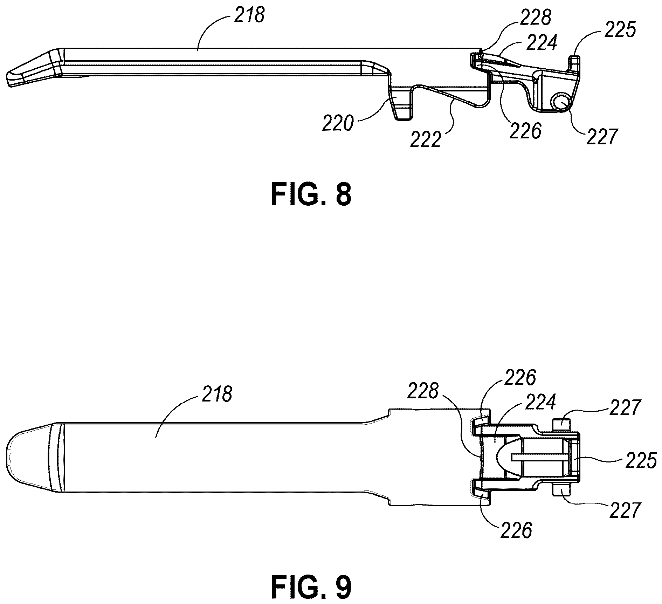

FIGS. 8-9 show pivotable anvil (218) in more detail. Pivotable anvil (218) comprises a central ramp (224), upper side ramps (226), and extensions (220). Central ramp (224) is positioned centrally on the top surface of a proximal portion of anvil (218). Central ramp (224) slopes downwardly in the proximal direction. A vertical surface (228) extends upwardly from a distal end of central ramp (224). As shown in FIG. 9, vertical surface (228) can have a curved profile. Side ramps (226) are positioned on either side of central ramp (224). A tab (225) extends upwardly from the proximal end of central ramp (224). Pins (227) extend outwardly from the proximal end of anvil (218) to engage cartridge jaw (216) such that anvil (218) is pivotable relative to cartridge jaw (216). While pins (227) of the present example pivot, pins (227) do not pivot about a fixed axis. Instead, pins (227) slide relative to cartridge jaw (216) in addition to pivoting, such that the pivot axis for anvil (218) slides relative to cartridge jaw (216).

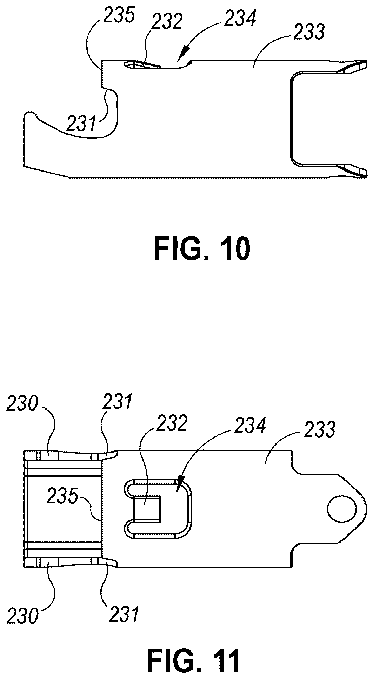

As shown in FIGS. 10-11, closure ring (233) comprises extensions (230), vertical surface (235), and tab (232). Tab (232) is positioned within a lateral hole or opening (234) that is formed through the sidewall of closure ring (233). Vertical surface (235) is positioned proximal of extensions (230) and is configured to engage vertical surface (228) of anvil (218). Vertical surface (235) extends around closure ring (233) and then proximally to form lower side surfaces (231). Opening (234) is configured to receive tab (225) of anvil (218). Tab (232) of closure ring (233) extends proximally within opening (234) and slopes downwardly.

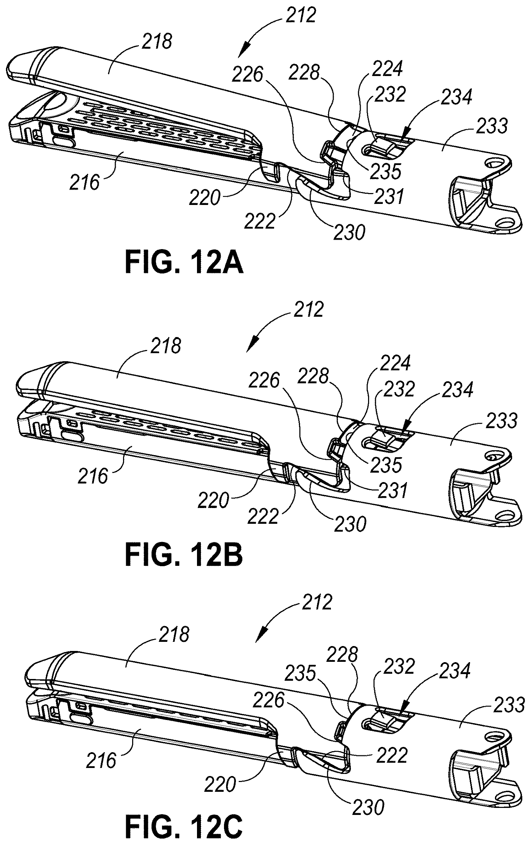

In an illustrative use, instrument (10) may be inserted to a surgical site in a nonarticulated state, with jaws (216, 218) closed (also referred to as a closed configuration). Once articulation joint (32) and end effector (212) are inserted to the desired site within the patient, anvil (218) may be pivoted away from cartridge jaw (216), as described below, to the open jaws (216, 218) to the position shown in FIG. 7 (an open configuration) such that jaws (216, 218) may be positioned about tissue. Articulation joint (32) may be remotely articulated an articulation control, such that end effector (212) may be deflected to a desired angle (α). Alternatively, end effector (212) may be articulated at articulation joint (32) prior to opening jaws (216, 218). Closure trigger (26) may then be actuated toward pistol grip (24) to cause the closing of anvil (218) toward cartridge jaw (216), as shown in FIGS. 12A-12C. Such closing of anvil (218) is provided through a closure tube (34) and closure ring (233), which both longitudinally translate relative to handle assembly (20) and cartridge jaw (216) in response to pivoting of closure trigger (26) relative to pistol grip (24). Articulation joint (32) is operable to communicate longitudinal movement from closure tube (34) to closure ring (233).

As closure ring (233) translates distally in response to advancement of closure tube (34), closure ring (233) translates relative to anvil (218) to engage anvil (218). As shown in FIGS. 12A-12B, vertical surface (235) of closure ring (233) engages central ramp (224) of anvil (218) at the open configuration. As closure ring (233) translates distally, closure ring (233) cams along central ramp (224) of anvil (218) to pivot anvil (218) toward cartridge jaw (216). FIG. 12C shows anvil (218) fully closed relative to cartridge jaw (216) (the closed configuration). At full closure, vertical surface (235) of closure ring (233) contacts vertical surface (228) of anvil (218).

### II. Improved End Effector Closure Features

As described above, closure tube (34) translatably actuates closure ring (233) relative to cartridge jaw (216) and anvil jaw (218), which pivotably drives anvil jaw (218) relative to cartridge jaw (216). As also discussed above, such configurations may undesirably require relatively high user input forces to advance the anvil jaw through an initial portion of its closure stroke, when the end effector closure components are relatively less mechanically advantaged due to the relatively larger angle between the anvil jaw and the cartidge jaw. To minimize these requisite input forces and the resulting internal stresses experienced by the end effector closure components, it may be desirable alternatively configure the end effector closure components so that the anvil jaw closes initially at a slower closure rate, when the end effector closure components are relatively less mechanically advantaged, and subsequently at a faster closure rate, when the end effector closure components are relatively more mechanically advantaged.

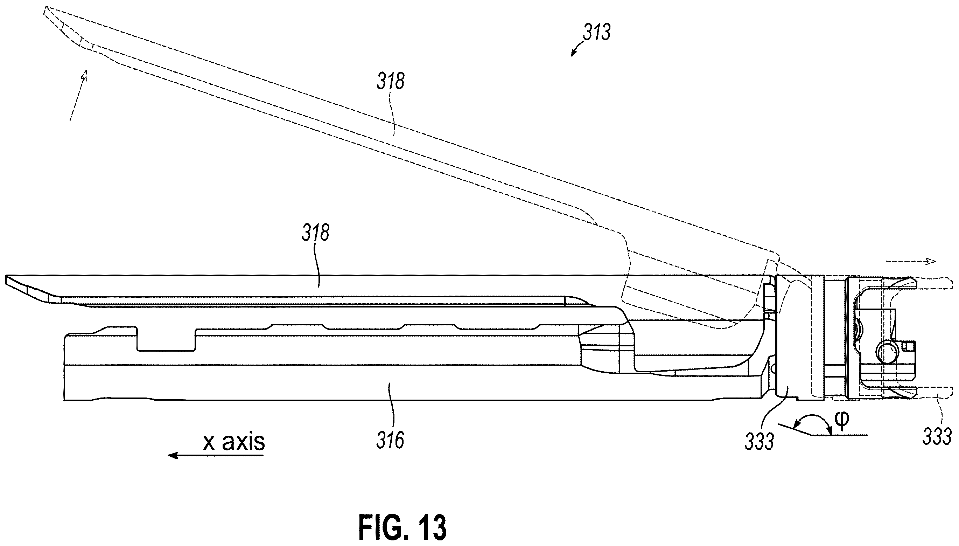

FIG. 13 shows an illustrative end effector (312) that may be readily incorporated into instrument (10) and is advantageously configured in the manner presented above. End effector (312) may be substantially similar in form and function to end effectors (40, 213), except as otherwise described below. End effector (312) comprises a cartridge jaw (316) (also referred to as a cartridge jaw), a pivotable anvil (318) (also referred to as an anvil jaw), and a translatable cam in the form of a closure ring (333). Cartridge jaw (316) is similar to cartridge jaws (42, 216) of end effectors (40, 313). Pivotable anvil (318) is similar to anvil jaws (44, 218), except that pivotable anvil (318) comprises channels (350) and a uniquely contoured ramped surface (352) that engage closure ring (333) to optimize closure of end effector (312) in the manner described above, and to reduce certain relative rotations between closure ring (333) and end effector jaws (316, 318). Closure ring (333) of end effector (312) is similar to closure rings (36, 233) except as otherwise described below.

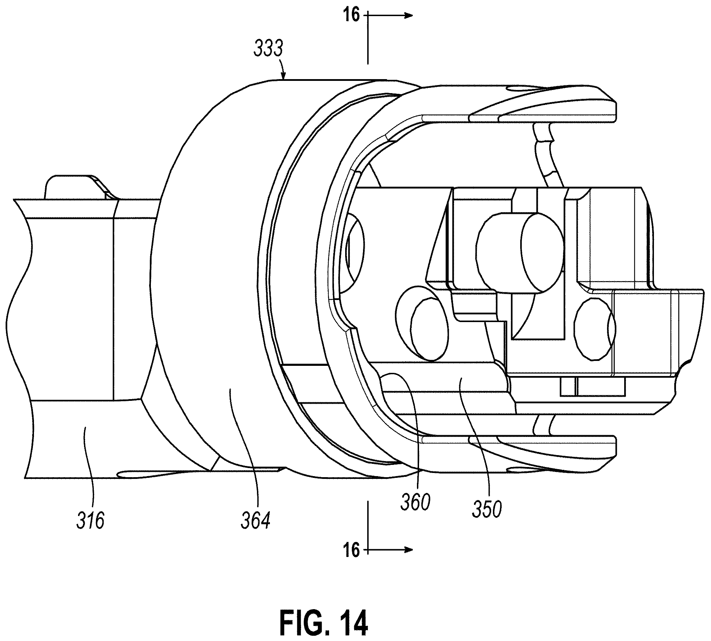

As shown, closure ring (333) is translatable relative to cartridge jaw (316) along an x-axis to thereby transition pivotable anvil (318) between the open configuration and the closed configuration. As shown in FIG. 14, lateral sides of closure ring (333) include protrusions (360) (also referred to as bumps) which translate longitudinally within respective channels (350) on lateral sides of cartridge jaw (316). Protrusions (360) extend longitudinally along at least a portion of closure ring body (364) such that they are capable of traveling a length within channels (350). As will be appreciated by one skilled in the art, protrusions (360) residing within channels (350) will prevent or inhibit a rotation of closure ring (333) about the x-axis and relative to the cartridge jaw (316) and the anvil jaw (318). Prevention or inhibition of rotation of closure ring (333) by protrusions (360) can occur at any location of translation of closure ring (333) since protrusions (360) remain in respective channels (350) throughout the entire length of translation.

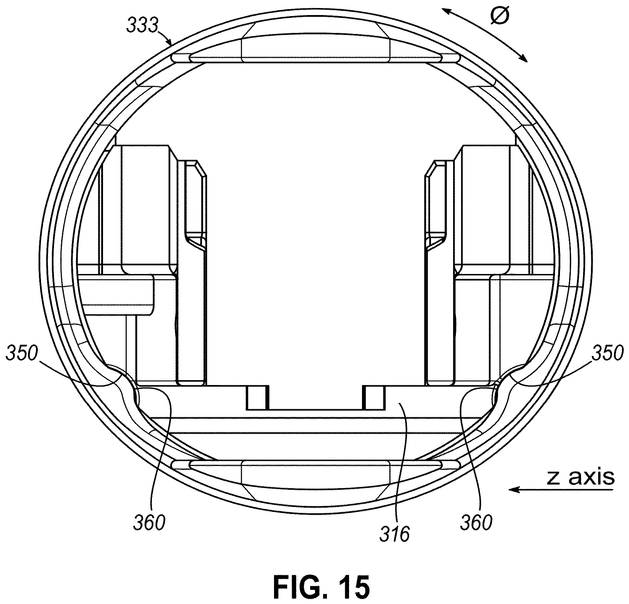

As best shown in FIG. 15, closure ring (333) includes two protrusions (360) positioned within respective channels (350) of cartridge jaw (316). Importantly, while two protrusions (360) and channels (350) are shown, one or more of each may still be operable to prevent or inhibit rotation. As shown, each channel (350) and protrusion (360) are laterally opposed to one another. As described above, channels (350) and protrusions (360) may be operable to prevent or inhibit a rotation (Θ) (see FIG. 15) about the X-Axis and they may also be operable to prevent or inhibit a rotation (φ) (see FIG. 13) about the Z-Axis (see FIG. 15). As one skilled in the art will appreciate, the longitudinally longer protrusions (360) are, the smaller the permitted rotations (Θ) and (φ) will be. As rotations (Θ) and (φ) are minimized, so is the tolerance between closure ring (333) and anvil jaw (318), thereby ensuring precise closure of anvil jaw (318). While protrusions (360) are shown on closure ring (333) and channels (350) are shown on cartridge jaw (316), these positions may be reversed wholly or partially such that some or all of channels (350) are positioned on closure ring (333) and some or all of protrusions (360) are positioned on cartridge jaw (316).

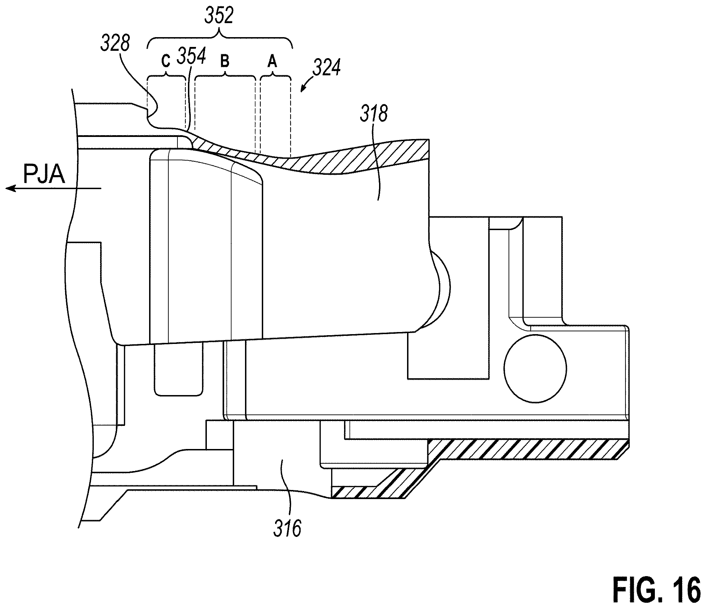

As such, FIGS. 16-17C illustrate the interaction between closure ring (333) and anvil jaw (318) during distal translation of closure ring (333). A proximal end of anvil jaw (318) includes a central ramp (324) having a ramped surface (352) on an upper side of anvil jaw (318). Ramped surface (352) is configured to cammingly engage closure ring (333) and may be defined as having a proximal engagement portion (A), which is proximal to an intermediate engagement portion (B), which is proximal to a distal engagement portion (C). A contact surface (362) of closure ring (333) contacts each portion of ramped surface (352) to thereby transition end effector (313) from the open configuration to the closed configuration. Engagement portions (A, B, C) are successively longitudinally positioned relative to one another and are smoothly blended so as to be free of any adjoining planar surfaces that are angled relative to one another. As will be described below, intermediate engagement portion (B) defines a concave curve and distal engagement portion (C) defines a convex curve and includes a radius which may be the same as a radius of contact surface (352).

Proximal engagement portion (A) is nearest to the pivot point of anvil jaw (318) such that it is the lowest mechanically advantaged region which results in the most angular movement of anvil jaw (318) given a certain distal translation distance of closure ring (333). As the geometries of engagement portions (A, B, C) indicate, initial angular movement of anvil jaw (318) is decreased from previously known anvil jaws to thereby wait until contact surface (362) is further from the pivot point of anvil jaw (318) before there is substantial anvil jaw (318) movement such that angular rotation is increased once the mechanical advantage has increased.

A slope of ramped surface (352) gradually increases from proximal engagement portion (A) to intermediate engagement portion (B) such that there is a slow closure rate of anvil jaw (318). The slope then rapidly increases through a distal portion of intermediate engagement portion (B) and into distal engagement portion (C) such that there is a more rapid closure rate of anvil jaw (318). Slope may then approach and reach a zero slope at a distal portion of distal engagement portion (C) such that the closure rate of anvil jaw (318) goes to zero. Slope of ramped surface (352) may be relative to a pivot jaw axis (PJA), described in more detail below.

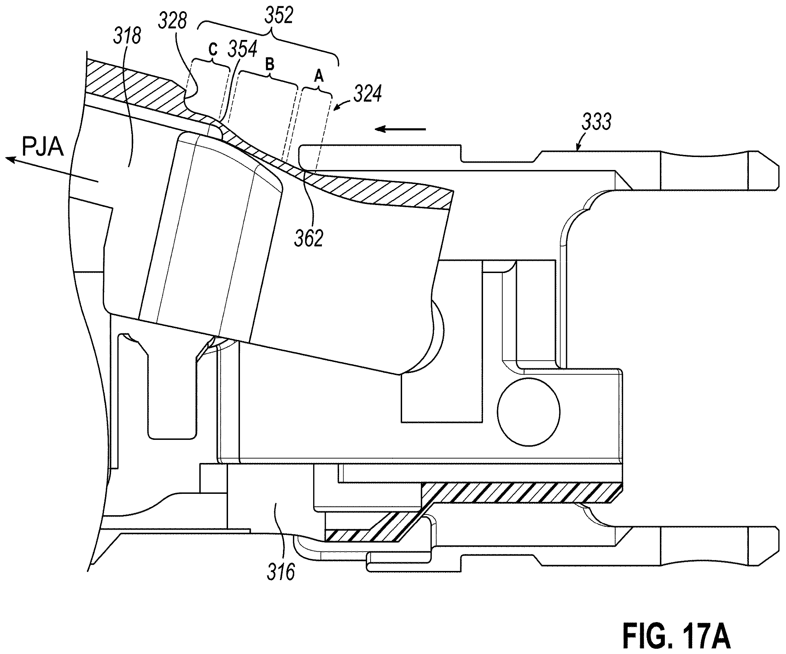

As shown in FIG. 17A with end effector (313) in the open configuration, contact surface (362) begins by translating distally along proximal engagement portion (A) towards intermediate engagement portion (B). Pivot jaw (318) may define a pivot jaw axis (PJA) along a longitudinal length, as shown in FIG. 16. Proximal engagement portion (A) may be inclined at an angle of less than or equal to 10 degrees relative to the pivot jaw axis (PJA). Accordingly, when contact surface (362) translates distally along proximal engagement portion (A), minimal closure of anvil jaw (318) may occur as compared to closure achieved along the intermediate and distal engagement portions (B, C).

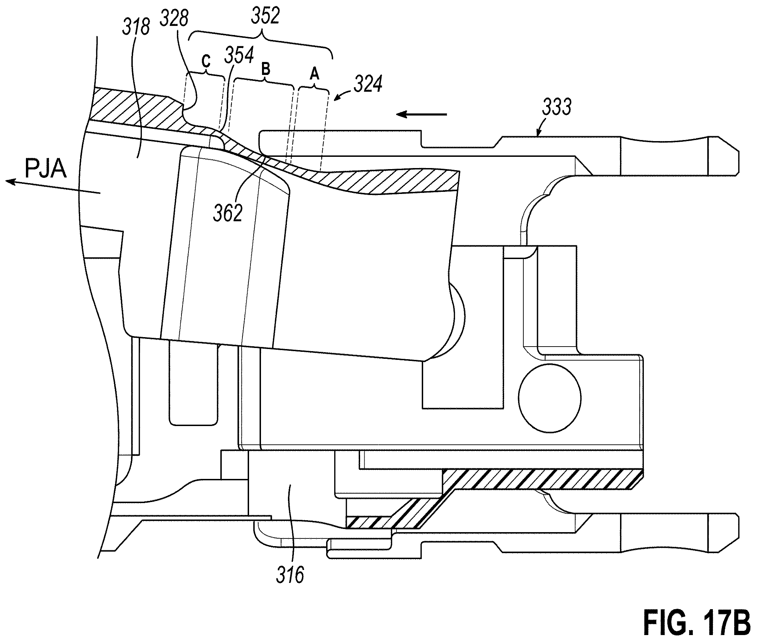

As shown in FIG. 17B, proximal engagement portion (B) defines a concave curve such that the slope of ramped surface (352) and the resulting angular rate of rotation of anvil jaw (318) toward cartidge jaw (316) progressively increase as contact surface (362) translates from a proximal portion to a distal portion of intermediate engagement portion (B). Stated another way, the angle relative to pivot jaw axis (PJA) of intermediate engagement portion (B) increases in a proximal to distal direction. Accordingly, a mechanical advantage for closing end effector (313) is greater when contact surface (362) is at the proximal portion than at the distal portion of intermediate engagement portion (B). An inflection point (354) is present at the intersection of intermediate engagement portion (B) and distal engagement portion (C). Inflection point (354) occurs when a distal end of the concave curve of intermediate engagement portion (B) meets a proximal end of the convex curve of distal engagement portion (C). Inflection point (354) defines a location of greatest angle of ramped surface (352) relative to pivot jaw axis (PJA) and may be located at approximately 80% to approximately 85% of a total travel distance of closure ring (333) along ramped surface (352), measured from a proximal end of ramped surface (352). This total translation length may be interpreted as a straight-line travel distance parallel to the pivot jaw axis (PJA) or as a total travel length along ramped surface (352). While inflection point (354) has been depicted as a singular point, it may also be a plane of constant slope.

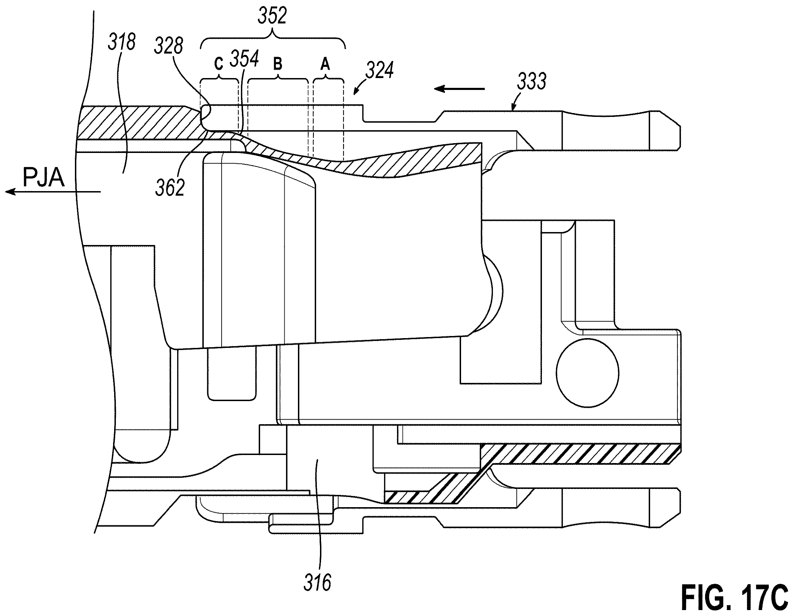

FIG. 17C shows end effector (313) in the closed configuration when closure ring (333) is distally located such that contact surface (362) rests against vertical surface (328) of anvil jaw (318) and where contact surface (362) nests within the radius defined by distal engagement portion (C). Vertical surface (328) may act as and be referred to as a stop.

As shown throughout FIGS. 17A-17C, contact surface (362) of closure ring (333) is radiused such that different portions of contact surface (362) will contact ramped surface (352) as contact surface (362) translates over ramped surface (352). Accordingly, contact surface (362) may include a constant radius, an increasing radius, or a decreasing radius. An increasing radius may increase in a direction towards an upper portion of closure ring (333), while a decreasing radius may do the opposite.

### III. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A first jaw (318) for use with an end effector (313) of a surgical instrument, a proximal end of the first jaw (318) including a ramp surface (352) comprising: a proximal engagement portion (A); an intermediate engagement portion (B), the intermediate engagement portion being distal to the proximal engagement portion, at least a portion of the proximal engagement portion being concavely curved; and a distal engagement portion (C) being distal to the intermediate engagement portion; wherein the ramp surface (352) is configured to be engaged by a translatable cam (333) of the surgical instrument to thereby pivot the first jaw (318) relative to a second jaw (316) of the end effector (313), the first (318) and second jaws (316) configured to cooperate to clamp and staple tissue (T) positioned therebetween.

### Example 2

The first jaw of example 1, the proximal engagement portion smoothly blending with the intermediate engagement portion, the intermediate engagement portion smoothly blending with the distal engagement portion.

### Example 3

The first jaw of any one of examples 1 and 2, the proximal engagement portion defining a first slope, the intermediate engagement portion defining a second slope that is larger than the first slope.

### Example 4

The first jaw of any one of examples 1-3, the distal engagement portion defining a third slope, the intermediate engagement portion defining a second slope that is smaller than the third slope.

### Example 5

The first jaw of any one of examples 1-4, at least a portion of the ramp surface being convexly curved to thereby define an inflection point (354) between the intermediate engagement portion and the distal engagement portion, the inflection point being positioned distal to a proximal most point of the ramp surface by at least 80 percent of a whole length of the ramp surface.

### Example 6

A surgical instrument comprising: an end effector including the first jaw of any one of examples 1-5 and a second jaw (316) coupled to the first jaw; and a translatable cam (333) translatable distally to cammingly engage the ramp surface of the first jaw and thereby drive the first jaw from an open position toward a closed position.

### Example 7

The surgical instrument of example 6, the translatable cam being configured to contact each of the proximal engagement portion, the intermediate engagement portion, and the distal engagement portion sequentially to thereby pivot the first jaw relative to the second jaw.

### Example 8

The surgical instrument of any one of examples 6-7, the surgical instrument further including a shaft proximal to the end effector and defining a longitudinal axis, the translatable cam being configured to translate parallel to the longitudinal axis, a distal portion of the distal engagement portion including a stop (328) configured to engage the translatable cam.

### Example 9

The surgical instrument of example 8, the stop being substantially perpendicular to the longitudinal axis when the translatable cam is engaged with the stop.

### Example 10

The surgical instrument of any one of examples 6-9, the translatable cam defining a contact surface (362) configured to contact each of the proximal engagement portion, the intermediate engagement portion, and the distal engagement portion, the contact surface being defined by a radius such that the contact surface nests within the distal engagement portion.

### Example 11

The surgical instrument of any one of examples 6-10, the intermediate engagement portion including a first length and a second length, the first length and the second length being of equal distance, the first length being proximal to the second length, the translatable cam being configured to slide along the first length to thereby pivot the first jaw relative to the second jaw by a first angle, the translatable cam being further configured to slide along the second length to thereby pivot the first jaw relative to the second jaw by a second angle, the first angle being less than the second angle.

### Example 12

The surgical instrument of any one of examples 6-11, the second jaw including channels (350) on lateral sides thereof, the translatable cam including protrusions (360) on lateral sides thereof that are slidable within the channels.

### Example 13

The surgical instrument of example 12, the second jaw defining a longitudinal axis, the protrusions and the channels being configured to cooperate to inhibit rocking of the translatable cam relative to second jaw about an axis perpendicular to the longitudinal axis.

### Example 14

The surgical instrument of any one of examples 12-13, the second jaw defining a longitudinal axis, the protrusions and the channels being configured to cooperate to inhibit rotation of the translatable cam relative to the second jaw about the longitudinal axis.

### Example 15

The surgical instrument of any one of examples 12-14, the channels extending longitudinally beyond the protrusions.

The following clauses also relate to various non-exhaustive ways in which the teachings herein may be combined or applied.

### Clause 1

A first jaw for use with an end effector of a surgical instrument, a proximal end of the first jaw including a ramp surface comprising: a proximal engagement portion; an intermediate engagement portion, the intermediate engagement portion being distal to the proximal engagement portion, at least a portion of the proximal engagement portion being concavely curved; and a distal engagement portion being distal to the intermediate engagement portion; wherein the ramp surface is configured to be engaged by a translatable cam of the surgical instrument to thereby pivot the first jaw relative to a second jaw of the end effector, the first and second jaws configured to cooperate to clamp and staple tissue positioned therebetween.

### Clause 2

The first jaw of clause 1, the proximal engagement portion smoothly blending with the intermediate engagement portion, the intermediate engagement portion smoothly blending with the distal engagement portion.

### Clause 3

The first jaw of clause 1, the proximal engagement portion defining a first slope, the intermediate engagement portion defining a second slope that is larger than the first slope.

### Clause 4

The first jaw of clause 1, the distal engagement portion defining a third slope, the intermediate engagement portion defining a second slope that is smaller than the third slope.

### Clause 5

The first jaw of clause 1, at least a portion of the ramp surface being convexly curved to thereby define an inflection point between the intermediate engagement portion and the distal engagement portion, the inflection point being positioned distal to a proximal most point of the ramp surface by at least 80 percent of a whole length of the ramp surface.

### Clause 6

A surgical instrument comprising: an end effector including the first jaw of clause 1 and a second jaw coupled to the first jaw; and a translatable cam translatable distally to cammingly engage the ramp surface of the first jaw and thereby drive the first jaw from an open position toward a closed position.

### Clause 7

The surgical instrument of clause 6, the translatable cam being configured to contact each of the proximal engagement portion, the intermediate engagement portion, and the distal engagement portion sequentially to thereby pivot the first jaw relative to the second jaw.

### Clause 8

The surgical instrument of clause 7, the surgical instrument further including a shaft proximal to the end effector and defining a longitudinal axis, the translatable cam being configured to translate parallel to the longitudinal axis, a distal portion of the distal engagement portion including a stop configured to engage the translatable cam.

### Clause 9

The surgical instrument of clause 8, the stop being substantially perpendicular to the longitudinal axis when the translatable cam is engaged with the stop.

### Clause 10

The surgical instrument of clause 6, the translatable cam defining a contact surface configured to contact each of the proximal engagement portion, the intermediate engagement portion, and the distal engagement portion, the contact surface being defined by a radius such that the contact surface is configured to nest within a distal end of the distal engagement portion.

### Clause 11

The surgical instrument of clause 6, the intermediate engagement portion including a first length and a second length, the first length and the second length being of equal distance, the first length being proximal to the second length, the translatable cam being configured to slide along the first length to thereby pivot the first jaw relative to the second jaw by a first angle, the translatable cam being further configured to slide along the second length to thereby pivot the first jaw relative to the second jaw by a second angle, the first angle being less than the second angle.

### Clause 12

The surgical instrument of clause 6, the second jaw including channels on lateral sides thereof, the translatable cam including projections on lateral sides thereof that are slidable within the channels.

### Clause 13

The surgical instrument of clause 12, the second jaw defining a longitudinal axis, the projections and the channels being configured to cooperate to inhibit rocking of the translatable cam relative to second jaw about an axis perpendicular to the longitudinal axis.

### Clause 14

The surgical instrument of clause 12, the second jaw defining a longitudinal axis, the projections and the channels being configured to cooperate to inhibit rotation of the translatable cam relative to the second jaw about the longitudinal axis.

### Clause 15

The surgical instrument of clause 12, the channels extending longitudinally beyond the projections.

### Clause 16

A surgical instrument comprising an end effector including the first jaw of clause 1 and a second jaw coupled to the first jaw, wherein one of the first jaw or the second jaw comprises an anvil having a plurality of pockets configured to form staples ejected by the other of the first jaw or the second jaw.

### Clause 17

An end effector for use with a surgical instrument, the end effector comprising: a first jaw including a first jaw body and at least one channel or at least one projection disposed on a lateral side of the first jaw body; a second jaw pivotable relative to the first jaw; and a translatable cam operatively coupled with the first jaw and the second jaw and including the other of at least one channel or at least one projection, the translatable cam being translatable relative to the first jaw such that the at least one projection is translatable within the at least one channel, the translatable cam being configured to cammingly engage the second jaw as the translatable cam translates distally relative to the first jaw to thereby pivot the second jaw relative to the first jaw, the at least one channel and the least one projection being configured to cooperate to inhibit rotation of the translatable cam relative to the first and second jaws about a longitudinal axis of the end effector.

### Clause 18

The end effector of clause 17, the first jaw defining the longitudinal axis, the translatable cam being translatable parallel to the longitudinal axis, the at least one projection being configured to inhibit rotation of the translatable cam about a first axis perpendicular to the longitudinal axis.

### Clause 19

The end effector of clause 18, the second jaw defining a pivot axis about which the second jaw is pivotable relative to the first jaw, the first axis being parallel to the pivot axis.

### Clause 20

A method of using a surgical instrument including an end effector having a first jaw, a second jaw, and a translatable ring, the method comprising: advancing the translatable ring a first distance along the first jaw and the second jaw to thereby pivot a distal end of the first jaw towards a distal end of the second jaw at a first rate; and advancing the translatable ring a second distance along the first jaw and the second jaw to thereby pivot the distal end of the first jaw towards the distal end of the second jaw at a second rate; the first distance being proximal to the second distance, the first rate being slower than the second rate.

### IV. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as those made available by Auris Health, Inc. of Redwood City, CA or by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A first jaw (318) for use with an end effector (313) of a surgical instrument, a proximal end of the first jaw (318) including a ramp surface (352) comprising:
(a) a proximal engagement portion (A);
(b) an intermediate engagement portion (B), the intermediate engagement portion being distal to the proximal engagement portion, at least a portion of the proximal engagement portion being concavely curved; and
(c) a distal engagement portion (C) being distal to the intermediate engagement portion;
wherein the ramp surface (352) is configured to be engaged by a translatable cam (333) of the surgical instrument to thereby pivot the first jaw (318) relative to a second jaw (316) of the end effector (313), the first (318) and second jaws (316) configured to cooperate to clamp and staple tissue (T) positioned therebetween.

2. The first jaw of claim 1, the proximal engagement portion smoothly blending with the intermediate engagement portion, the intermediate engagement portion smoothly blending with the distal engagement portion.

3. The first jaw of any one of claims 1 and 2, the proximal engagement portion defining a first slope, the intermediate engagement portion defining a second slope that is larger than the first slope.

4. The first jaw of any one of claims 1-3, the distal engagement portion defining a third slope, the intermediate engagement portion defining a second slope that is smaller than the third slope.

5. The first jaw of any one of claims 1-4, at least a portion of the ramp surface being convexly curved to thereby define an inflection point (354) between the intermediate engagement portion and the distal engagement portion, the inflection point being positioned distal to a proximal most point of the ramp surface by at least 80 percent of a whole length of the ramp surface.

6. A surgical instrument comprising:
(a) an end effector including the first jaw of any one of claims 1-5 and a second jaw (316) coupled to the first jaw; and
(b) a translatable cam (333) translatable distally to cammingly engage the ramp surface of the first jaw and thereby drive the first jaw from an open position toward a closed position.

7. The surgical instrument of claim 6, the translatable cam being configured to contact each of the proximal engagement portion, the intermediate engagement portion, and the distal engagement portion sequentially to thereby pivot the first jaw relative to the second jaw.

8. The surgical instrument of any one of claims 6-7, the surgical instrument further including a shaft proximal to the end effector and defining a longitudinal axis, the translatable cam being configured to translate parallel to the longitudinal axis, a distal portion of the distal engagement portion including a stop (328) configured to engage the translatable cam.

9. The surgical instrument of claim 8, the stop being substantially perpendicular to the longitudinal axis when the translatable cam is engaged with the stop.

10. The surgical instrument of any one of claims 6-9, the translatable cam defining a contact surface (362) configured to contact each of the proximal engagement portion, the intermediate engagement portion, and the distal engagement portion, the contact surface being defined by a radius such that the contact surface nests within the distal engagement portion.

11. The surgical instrument of any one of claims 6-10, the intermediate engagement portion including a first length and a second length, the first length and the second length being of equal distance, the first length being proximal to the second length, the translatable cam being configured to slide along the first length to thereby pivot the first jaw relative to the second jaw by a first angle, the translatable cam being further configured to slide along the second length to thereby pivot the first jaw relative to the second jaw by a second angle, the first angle being less than the second angle.

12. The surgical instrument of any one of claims 6-11, the second jaw including channels (350) on lateral sides thereof, the translatable cam including protrusions (360) on lateral sides thereof that are slidable within the channels.

13. The surgical instrument of claim 12, the second jaw defining a longitudinal axis, the protrusions and the channels being configured to cooperate to inhibit rocking of the translatable cam relative to second jaw about an axis perpendicular to the longitudinal axis.

14. The surgical instrument of any one of claims 12-13, the second jaw defining a longitudinal axis, the protrusions and the channels being configured to cooperate to inhibit rotation of the translatable cam relative to the second jaw about the longitudinal axis.

15. The surgical instrument of any one of claims 12-14, the channels extending longitudinally beyond the protrusions.
